# EUROPEAN PATENT SPECIFICATION

(11) **EP 2 632 418 B1**
(45) Date of publication and mention of the grant of the patent: **31.12.2014**
(21) Application number: 11764193.6
(22) Date of filing: 04.10.2011
(51) Int. Cl.: A61K 8/35, A61Q 17/04, A61K 8/37

(54) **A STABLE SUNSCREEN COMPOSITION**
STABILE SONNENSCHUTZZUSAMMENSETZUNG
COMPOSITION PHOTOPROTECTRICE STABLE

(30) Priority: 12.01.2011 EP 11150730; 25.10.2010 IN MU29512010
(43) Date of publication of application: 04.09.2013
(73) Proprietor: Unilever N.V., 3013 AL Rotterdam (NL); Unilever PLC, London, Greater London EC4P 0DY (GB)
(72) Inventor: CHAVAN, Mohan Vijaykumar, Bangalore 560 066 (IN); KUNJUPILLAI, Balu, Bangalore 560 066 (IN); VAIDYA, Ashish, Anant, Bangalore 560 066 (IN)
(74) Representative: Corsten, Michael Allan
(86) International application number: PCT/EP2011/067279
(87) International publication number: WO 2012/055678

(56) References cited:
- WO-A1-2008/022946
- WO-A1-2011/042358
- WO-A2-2005/094772
- WO-A2-2011/063864
- WO-A2-2011/063865
- JP-A- 2009 196 980
- US-A- 6 024 944
- US-A1- 2009 220 625
- Michael Starch ET AL: "Expanding Silicone Technologies for Sun Care: Performance Complements Aesthetics", , 12 July 2008 (2008-07-12), pages 1-15, XP55022770, Retrieved from the Internet: URL:http://web.archive.org/web/20081207060 312/http://www.dowcorning.com/ko_KR/conten t/publishedlit/27-1303.pdf [retrieved on 2012-03-23]
- IBRAHIM HANNO ET AL: "Polyamide Nanocapsules and Nano-emulsions Containing ParsolÂ TM MCX and ParsolÂ TM 1789:Release,Skin Penetration and Photo-Stability Studies", PHARMACEUTICAL RESEARCH, KLUWER ACADEMIC PUBLISHERS-PLENUM PUBLISHERS, NL, vol. 29, no. 2, 23 September 2011 (2011-09-23), pages 559-573, XP035005693, ISSN: 1573-904X, DOI: 10.1007/S11095-011-0592-5
- AESCHBACH R ET AL: "Antioxidant actions of thymol, carvacrol, 6-gingerol, zingerone and hydroxytyrosol", FOOD AND CHEMICAL TOXICOLOGY, PERGAMON, GB, vol. 32, no. 1, 1 January 1994 (1994-01-01), pages 31-36, XP023863355, ISSN: 0278-6915, DOI: DOI:10.1016/0278-6915(84)90033-4 [retrieved on 1994-01-01]
- Fernandez X. et al.: "Chemical composition of absolute and supercritical carbon dioxide extract of Aframomom melegueta", Flavour Fragrance Journal, vol. 21 8 August 2008 (2008-08-08), pages 162-165, XP002641538, Retrieved from the Internet: URL:http://onlinelibrary.wiley.com/doi/10. 1002/ffj.1554/pdf [retrieved on 2011-06-14]

## Description

### Technical field

The present invention relates to a personal care composition. Particularly, the invention relates to a stable sunscreen composition.

### Background of the Invention

Consumers from all over the world want to have good appearance of their skin. One of the ways that they do this is by protecting their skin from sunlight especially the harmful ultraviolet rays emitted by the sun. They often achieve this through use of personal care compositions e.g. sunscreen compositions.

Sunscreen compositions usually contain sunscreens/sunblocks. Sunblocks help to protect the skin against possible irritation and sunburn caused by solar radiation. Sunblocks generally are inorganic particulates like zinc oxide or titania that reflect the incident UV-visible rays thus protecting the skin. Sunscreens are generally organic molecules that absorb the UV radiation and emit the energy in a different form, for instance in the visible range of the spectrum or in the form of heat, thereby protecting the skin against irritation and sunburn. Sunscreens are generally small organic molecules.

One commonly used organic sunscreen is a dibenzoylmethane compound specifically 4-t-butyl-4'-methoxydibenzoylmethane sold as Parsol 1789™ (or Avobenzone™). The problem associated with this sunscreen is its relatively unstable nature, especially in the presence of a UV-B sunscreen of the p-methoxycinnamic acid group. Efforts have been made to add some external molecule, like Octocrylene™, to improve stability of dibenzoylmethane compounds in personal care compositions. Yet there is a need for further improvement in stability of Parsol 1789™ in the presence of the p-methoxycinnamic acid based UV-B suncreen. Further, there is a need for using more readily and easily available compound as a sunscreen stabilizer thereby ensuring reduced cost of such sunscreen compositions. The present inventors have worked intensively on solving this problem and found that a derivative of an active (gingerol) found in ginger i.e. zingerone, provides for such improved stability.

There are some publications that relate to inclusion of ginger extracts and gingerol in skin care compositions. WO 2010/000877 discloses inclusion of actives from ginger (paradol, gingerols and shogaols) as anti-inflammatory agents for skin care. US 2008/069784 discloses personal care compositions containing an active selected from the group consisting of ginger extract containing following chemical structures - 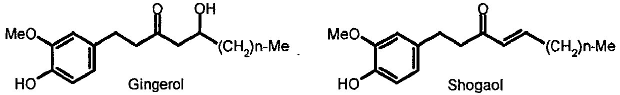 along with sunscreens. JP 2009 196980 A discloses use of zingerone 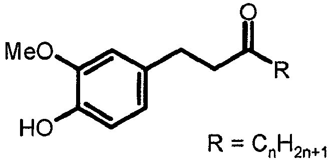 as the melanin formation inhibitor and beautification cosmetic but does not disclose any combination with UVA sunscreens. WO 2011/042358 discloses fluid compositions for protecting the skin against UV radiation, characterized by high protection factor, but does not disclose a UVB organic sunscreen of the p-methoxycinnamic acid tyre and a weight ratio of zingerone to dibenzoylmethane from 0.8 to 2.0.

It is thus an object of the present invention to provide a personal care composition with relatively stabilized UVA sunscreen dibenzoylmethane or its derivative when present along with a UV-B sunscreen of the p-methoxycinnamic acid group. It is yet another object of the present invention to stabilize dibenzoylmethane or its derivative though use of derivatives of natural materials. It is yet another object of the present invention to stabilize dibenzoyl methane based UV-A sunscreen in the presence of p-methoxycinnamic acid UV-B sunscreen when present in fatty acid or silicone elastomer based compositions.

### Summary of the invention:

Accordingly the present invention provides for a photostable sunscreen composition comprising,
(a) 0.1 % to 10 % by weight dibenzoylmethane or its derivative;
(b) 0.1 % to 10 % by weight p-methoxycinnamic acid or its derivative;
(c) 0.5 to 5 % by weight zingerone; and
(d) a cosmetically acceptable base comprising silicone elastomer
wherein weight ratio of zingerone to dibenzoylmethane is from 0.8 to 2.0.

The present invention also provides for use of the composition of the invention for photoprotection of human keratinous substrates.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of". It is noted that the examples given in the description below are intended to clarify the invention and are not intended to limit the invention to those examples per se. Similarly, all percentages are weight/weight percentages unless otherwise indicated. All amounts are by weight of the final composition, unless otherwise specified. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The disclosure of the invention as found herein is to be considered to cover all embodiments as found in the claims as being multiply dependent upon each other irrespective of the fact that claims may be found without multiple dependency or redundancy.
"Personal Care Composition" as used herein, is meant to include a composition for topical application to skin and/or hair of mammals, especially humans. Such a composition may be generally classified as leave-on or rinse off, and includes any product applied to a human body for basically sunscreen benefits but may also have additional benefits like improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of personal care compositions include leave-on skin lotions and creams, shampoos, conditioners, shower gels, toilet bars, antiperspirants, deodorants, lipsticks, foundations, mascara, sunless tanners and sunscreen lotions.

"Skin" as used herein is meant to include skin on the face and body especially the generally sun exposed parts of the body e.g. neck, arms, hands, legs, and scalp but may also include regions of the body which are intentionally exposed e.g. on the beach like the chest, back, underarms and buttocks. The composition of the invention is also of relevance to applications on any other keratinous substrate of the human body other than skin e.g. hair where products may be formulated with specific aim of providing photoprotection.

The ultraviolet (UV) portion of solar radiation is divided into three ranges based on wavelength viz. UVC (200-280 nm), UVB (280-320 nm) and UVA (320 - 400 nm). In a photoprotection formulation people generally use both UVA and UVB sunscreen as most of the UVC gets absorbed by the ozone layer.

As per the present invention, the personal care composition comprises a dibenzoylmethane or its derivative, p-methoxycinnamic acid or its derivative, zingerone and a cosmetically acceptable base comprising silicone elastomer. 4-t-butyl-4'-methoxydibenzoylmethane is the preferred dibenzoylmethane derivatives for the purpose of the present invention.

4-t-butyl-4'-methoxydibenzoylmethane is one very well known dibenzoylmethane derivative which is a sunscreen agent sold under the trade names Avobenzone ™, Parsol 1789™, Eusolex 9020™, or Escalol 517™. Most of the consumer care products that intend to protect the substrate against UV radiation use a dibenzoylmethane compound. This is an oil soluble ingredient used in sunscreen products to provide protection against the full spectrum of UVA rays. 4-t-butyl-4'-methoxydibenzoylmethane exists in the ground state as a mixture of the enol and keto forms, favoring the chelated enol. It is able to absorb ultraviolet light over a wider range of wavelengths compared to many organic sunscreen agents. That is the reason it is called as "broad spectrum" sunscreen. 4-t-butyl-4'-methoxydibenzoylmethane has an absorption maximum of 357 nm.

According to the invention, the personal care composition preferably comprises 0.1 to 10 % dibenzoylmethane derivative, more preferably 0.1 to 4 % by weight of the composition.

The personal care composition of the invention comprises a UVB sunscreen para-methoxy cinnamic acid and its derivative e.g. 2-ethyl- hexyl 4-methoxycinnamate which is sold under the brand name of Parsol MCX™, to protect entire range of UV rays. According to the invention, the personal care composition comprises 0.1 to 10 %, preferably 0.1 to 6 %, most preferably 0.1 to 3 % ethylhexyl methoxycinnamate by weight of the composition. Inclusion of p-methoxycinnamic acid derivative is especially useful since in addition to providing the known UV-B protection, the personal care composition ensures better stability of dibenzoylmethane derivatives in the presence of p-methoxycinnamic acid derivative, with the use of zingerone.

The present invention is about the stabilization of dibenzoylmethane derivative by zingerone. The composition of the invention comprises 0.5 to 5 %, more preferably 1 to 3 %, and most preferably 1.2 to 2 % zingerone by weight of the composition. The chemical structure of zingerone is given below:

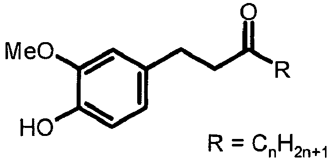

The value of n in the above structure is preferably in the range of 20 to 1, more preferably 5 to 1. Zingerone has the CAS Number 122-48-5. Zingerone is a widely available chemical but the examples in the present invention have been purchased from Ace Rasayan, Bangalore, India. The weight ratio of zingerone to dibenzoylmethane derivative is from 0.8:1 to 2.0:1. The optimum ratio of zingerone to dibenzoylmethane is from 0.9:1.1 to 1.1:0.9.

Zingerone may be prepared by derivatisation of active extracted from a natural material ginger or can be prepared by synthetic means. Extract of natural material ginger has gingerol which is thermally degraded to get zingerone. A typical process for preparation of zingerone is given below:

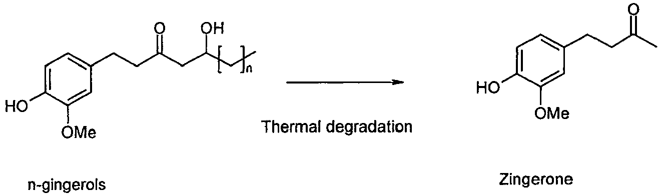

The personal care composition comprises a cosmetically acceptable base comprising silicone elastomer. The cosmetically acceptable base is preferably a cream, lotion, gel or emulsion.

Personal care compositions may be prepared using different cosmetically acceptable emulsifying or non-emulsifying systems and vehicles. When the cosmetically acceptable base comprises fatty acid, it is preferably present in 1 to 25 %, more preferably 3 to 25 %, further more preferably 5 to 25 % by weight of the composition. A more preferred format is a cream, further more preferably one which has a vanishing cream base. Vanishing cream base is one which comprises 5 to 25 % by weight fatty acid and 0.1 to 10 % by weight soap. In such creams, the fatty acid is preferably substantially a mixture of stearic acid and palmitic acid and the soap is preferably the potassium salt of the fatty acid mixture. The fatty acid in vanishing cream base is often prepared using hysteric acid which is substantially (generally about 90 to 95 %) a mixture of stearic acid and palmitic acid. A typical hysteric acid comprises about 52-55 % palmitic acid and 45-48 % stearic acid of the total palmitic-stearic mixture.

Thus, inclusion of hysteric acid and its soap to prepare the vanishing cream base is within the scope of the present invention. It is particularly preferred that the composition comprises higher than 7 %, preferably higher than 10 %, more preferably higher than 12 % by weight fatty acid.

The cosmetically acceptable base comprises silicone elastomer, it is preferably present in 10 to 60 % more preferably 20 to 50 % by weight of the composition.

Suitable silicone elastomers for use in the composition of the invention are DC 9045 a dimethicone crosspolymer commercially available from Dow Corning. Other useful silicone elastomer blends which may be used in the present invention are commercially available as
(i) DC 9027 (a blend of an ultra high viscosity dimethiconol and silicone elastomer in cyclopentasiloxane) available from Dow Corning
(ii) DC 9546 (blend of high molecular weight silicone elastomer, cyclopentasiloxane and a high molecular weight linear silicone polymer) available from Dow Corning,
(iii) EL8050 (a blend of high molecular weight polyglycol-modified silicone elastomer in isododecane) available from Dow Corning and
(iv) EL8051 (a blend of high molecular weight polyglycol-modified silicone elastomer in isodecyl neopentanoate) available from Dow Corning.

The cosmetically acceptable base is usually from 10 to 99.9 %, preferably from 50 to 99 % by weight of the composition. The cosmetically acceptable base include water or may be anhydrous. When the cosmetically acceptable base comprises fatty acid, water is preferably included in 35 to 90 %, more preferably 50 to 85 %, further more preferably 50 to 80 % by weight of the composition. When the cosmetically acceptable base comprises silicone elastomer, water may be included to prepare water-in-oil emulsion or the composition may comprise little or no water and so may be substantially anhydrous.

The composition of the invention may additionally comprise a skin lightening agent. The skin lightening agent is preferably chosen from a vitamin B3 compound or its derivative e.g. niacin, nicotinic acid, niacinamide or other well known skin lightening agents. Other vitamins like vitamin B6, vitamin B12, vitamin C, or vitamin A, may be included as skin lightening agents. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10 %, more preferably 0.2 to 5 % by weight of the composition.

Useful inorganic sun-blocks are also preferably used in the present invention. These include, for example, zinc oxide, iron oxide, silica, such as fumed silica, and titanium dioxide.

The composition according to the invention may also comprise other diluents. The diluents act as a dispersant or carrier for other materials present in the composition, so as to facilitate their distribution when the composition is applied to the skin.

Diluents other than water can include liquid or solid emollients, solvents, humectants, thickeners and powders.

The cosmetically acceptable base is usually from 10 to 99.9 %, preferably from 50 to 99 % by weight of the composition, and can, in the absence of other personal care adjuncts, form the balance of the composition.

The composition of the invention may comprise a conventional deodorant base as the cosmetically acceptable carrier. By a deodorant is meant a product in the stick, roll-on, or propellant medium which is used for personal deodorant benefit e.g. application in the under-arm area which may or may not contain antiperspirant actives.

Deodorant compositions can generally be in the form of firm solids, soft solids, gels, creams, and liquids and are dispensed using applicators appropriate to the physical characteristics of the composition. Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95 % and in many instances from 40 to 80 % by weight of the composition. Hydrophobic liquid carriers commonly can comprise one or more materials selected within the chemical classes of siloxanes, hydrocarbons, branched aliphatic alcohols, esters and ethers that have a melting point not higher than 25°C and a boiling point of at least 100°C. Hydrophilic carrier liquids that can be employed in compositions herein commonly comprise water and/or a mono or polyhydric alcohol or water-miscible homologue. Monohydric alcohols often are short chain, by which is meant that they contain up to 6 carbons, and in practice is most often ethanol or sometimes iso-propanol. Polyhydric alcohols commonly comprise ethylene or propylene glycol, or a homologue can be employed such as diethylene glycol. Other than this suitable other vehicle and component used for deodorant composition can be added.

The compositions of the present invention can comprise a wide range of other optional components which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The composition is formulated in any known format, more preferred formats being creams or lotions.

The invention will now be illustrated with the help of the following non-limiting examples.

### Examples

### Examples 1 and 2: Improvement in stability on inclusion of zingerone in a UV-A containing sunscreen composition

Photoprotective personal care compositions in a vanishing cream base were prepared as shown in table 1.

**Table 1**

| | Example 1 (wt %) | Example 2 (wt %) |
|---|---|---|
| Hysteric acid | 17 | 17 |
| Cetyl alcohol | 0.53 | 0.53 |
| Isopropyl myristate | 1.64 | 1.64 |
| Parsol 1789™ | 1.2 | 1.2 |
| Zingerone | - | 1.5 |
| Methyl paraben | 0.2 | 0.2 |
| Propyl paraben | 0.1 | 0.1 |
| Phenoxy ethanol | 4.4 | 4.4 |
| Glycerine | 1.0 | 1.0 |
| KOH (85 %) | 0.56 | 0.56 |
| Disodium EDTA | 0.04 | 0.04 |
| Dimethicone | 0.5 | 0.5 |
| Water | To 100 | To 100 |

### Quantification of Photostability of compositions

The compositions were applied (∼ 3 mg/cm²) on four clean glass plates to generate thin films of uniform thickness. Out of these, three plates were exposed to Atlas solar simulated radiations (UVA flux, 5.5 mW/cm²). One plate was removed after 30, 60 and 120 minutes of UV exposure, each. Fourth plate was kept un-exposed which served as the control sample. Subsequent to completion of the above protocol, all four films of the cream were separately dissolved in suitable HPLC grade methanol. The UV-A/BMDM (4-t-butyl, 4'-methoxydibenzoylmethane) quantification was done using Perkin Elmer UV/Visible Spectrometer. The absorbance at a scanning range of 200-800 nm was measured for each solution, using quartz cuvette, and respective blank solutions spectrometer. From the spectrum the % of UV-A compound i.e. Parsol-1789 remaining was read at various times (for a representative wavelength i.e. 355 nm) and summarized in table 2 below.

**Table 2**

| % UV-A remaining after | Example 1 | Example 2 |
|---|---|---|
| 30 minutes | 47 | 88 |
| 60 minutes | 26 | 56 |
| 120 minutes | 11 | 30 |

The data in table 2 indicates that composition as per the invention (example 2) provided improved photostability as compared to a conventional composition (example 1).

### Examples 3 to 5: Improvement in stability on inclusion of zingerone in a UV-A and UV-B containing sunscreen composition

Photoprotective personal care compositions in a vanishing cream base were prepared as shown in table 3.

**Table 3**

| | Example 3 (wt %) | Example 4 (wt %) | Example 5 (wt %) |
|---|---|---|---|
| Hystric acid | 17 | 17 | 17 |
| Cetyl alcohol | 0.53 | 0.53 | 0.53 |
| Isopropyl myristate | 1.64 | 1.64 | 1.64 |
| Parsol 1789™ | 1.2 | 1.2 | 1.2 |
| Parsol MCX™ | 2.25 | 2.25 | 2.25 |
| Octocrylene™ | - | - | 1.5 |
| Zingerone | - | 1.5 | - |
| Methyl paraben | 0.2 | 0.2 | 0.2 |
| Propyl paraben | 0.1 | 0.1 | 0.1 |
| Phenoxy ethanol | 4.4 | 4.4 | 4.4 |
| Glycerine | 1.0 | 1.0 | 1.0 |
| KOH (85 %) | 0.56 | 0.56 | 0.56 |
| Disodium EDTA | 0.04 | 0.04 | 0.04 |
| Dimethicone | 0.5 | 0.5 | 0.5 |
| Water | To 100 | To 100 | To 100 |

The photostability of the various compositions of table 3 was measured using the same procedure as used for examples 1 and 2 and are summarized in table 4 below:

**Table 4**

| % UV-A remaining after | Example 3 | Example 4 | Example 5 |
|---|---|---|---|
| 30 minutes | 62 | 70 | 55 |
| 60 minutes | 30 | 47 | 29 |
| 120 minutes | 21 | 44 | 12 |

The data in table 4 indicates that composition as per the invention (example 4 provided improved photostability as compared to a composition without zingerone (example 3) or a composition with a well known UV-A stabilizer, Octocrylene™ (example 5).

### Example 6 to 9: Improvement in stability on inclusion of zingerone in sunscreen compositions in a polymer base

Photoprotective personal care compositions in a polymer base were prepared as shown in table 5.

**Table 5**

| | Example 6 (wt %) | Example 7 (wt %) | Example 8 (wt %) | Example 9 (wt %) |
|---|---|---|---|---|
| Polymer EL 8051^{*} | 40 | 40 | 40 | 40 |
| Silicone 5225C^{**} | 10 | 10 | 10 | 10 |
| Isopropyl myristate | 6 | 6 | | 6 |
| Parsol 1789™ | 1.2 | 1.2 | 1.2 | 1.2 |
| Parsol MCX™ | - | - | 2.25 | 2.25 |
| Zingerone | - | 1.5 | - | 1.5 |
| Water | To 100 | To 100 | | To 100 |

| | | | | |
|---|---|---|---|---|
| * Polymer EL8051: is Isodecyl neopentanoate and dimethicone / bis isobutyl PPG-20 cross polymer available from Dow Corning. **Silicone 5225C is cyclopentasiloxane and PEG/PPG-18/18 dimethicone polymer available from Dow Corning. | | | | |

The photostability of the various compositions of table 5 was measured using the same procedure as used for examples 1 and 2 and are summarized in table 6 below:

**Table 6**

| % UV-A remaining after | Example 6 | Example 7 | Example 8 | Example 9 |
|---|---|---|---|---|
| 30 minutes | 51 | 78 | 71 | 67 |
| 60 minutes | 34 | 68 | 24 | 51 |
| 120 minutes | 18 | 38 | 9 | 23 |

The data in table 6 indicates that inclusion of zingerone in compositions as per the invention (example 7 and 9) provides for improved photostability of the respective compositions without zingerone (examples 6 and 8) when these are formulated in a polymer base.

### Example 10: Hair care composition

A hair care composition, as per the invention, as shown in table 7, was made in the form of a hair styling gel. Aristoflex AVC (cationic copolymer) was dissolved in water using a homogenizer. Parsol 1789™ and zingerone were separately dissolved in propylene carbonate. The above two mixtures were then mixed and homogenized by using a high speed homogenizer.

**Table 7**

| Ingredients | Percentage (weight) |
|---|---|
| Water | To 100 |
| Aristoflex AVC (cationic polymer) | 1.2 |
| Propylene carbonate (oil phase) | 9 |
| Parsol 1789TM | 1.2 |
| Zingerone | 1.2 |
| Methyl paraben | 0.2 |
| Propyl paraben | 0.1 |

### Examples 11 and 12: Effect of inclusion of ginger extract instead of zingerone

Compositions were prepared similar to that of example 4 except that the ingredient zingerone was replaced with ginger extract (0.1 % in example 11) and (1.5 % in example 12). The ginger extract used in examples 11 and 12 was sourced from Natural Remedies Pvt. Ltd., Bangalore, India and contained 3.0 % gingerol. The ginger extract was prepared by extracting the water extractable actives in water at a water: ginger ratio of 10:1. The extract was then concentrated and dried to a powder using a vacuum tray drier.

The photostability of the compositions example 11 and 12 was measured using a procedure similar to that used for measuring composition of example 4. The photostability of a sample of example 4 was repeated for comparison. The data is summarized in table 8 below:

**Table 8**

| | Example 11 | Example 12 | Example 4 |
|---|---|---|---|
| % UV-A remaining after 60 minutes | 18 | 22 | 45 |

The data in table 8 indicates that composition comprising zingerone (example 4) provides vastly improved stability of UVA sunscreen as compared to use of ginger extract (which contains gingerol).

The invention thus provides for a personal care composition with relatively stabilized UV-A sunscreen and this has been achieved through use of derivatives of natural materials.

## Claims

1. A photostable sunscreen composition comprising
(a) 0.1 % to 10 % by weight dibenzoylmethane or its derivative;
(b) 0.1 % to 10 % by weight p-methoxycinnamic acid or its derivative;
(c) 0.5 to 5 % by weight zingerone; and
(d) a cosmetically acceptable base comprising silicone elastomer;
wherein weight ratio of zingerone to dibenzoylmethane is from 0.8 to 2.0.

2. A composition as claimed in claim 1 wherein said dibenzoylmethane derivative is 4-tert-butyl-4'-methoxydibenzoylmethane.

3. A composition as claimed in claim 1 wherein said p-methoxycinnamic acid derivative is 2-ethyl-hexyl-4-methoxycinnamate.

4. A composition as claimed in any one of the preceding claims comprising 1.0 to 3 % zingerone by weight of the composition.

5. A composition as claimed in any one of the preceding claims wherein the cosmetically acceptable base is a cream, lotion, gel or emulsion.

6. Composition as claimed in any one of the preceding claims for use in a method of photoprotection of human keratinous substrates, the method comprising applying the composition to human keratinous substrates.

## Patentansprüche

1. Lichtbeständige Sonnenschutzzusammensetzung,
die Folgendes aufweist:
(a) 0,1 bis 10 Gew.-% Dibenzoylmethan oder ein Derivat davon;
(b) 0,1 bis 10 Gew.-% p-Methoxyzimtsäure oder ein Derivat davon;
(c) 0,5 bis 5 Gew.-% Zingeron; und
(d) einen kosmetisch akzeptablen Träger, der ein Siliconelastomer aufweist;
wobei das Gewichtsverhältnis zwischen Zingeron und Dibenzoylmethan 0,8 bis 2,0 beträgt.

2. Zusammensetzung nach Anspruch 1,
wobei das Dibenzoylmethan-Derivat 4-tert.-Butyl-4'-methoxydibenzoylmethan ist.

3. Zusammensetzung nach Anspruch 1,
wobei das p-Methoxyzimtsäure-Derivat 2-Ethylhexyl-4-methoxycinnamat ist.

4. Zusammensetzung nach einem der vorstehenden Ansprüche,
die 1,0 bis 3 % Zingeron aufweist, und zwar auf das Gewicht der Zusammensetzung bezogen.

5. Zusammensetzung nach einem der vorstehenden Ansprüche,
wobei der kosmetisch akzeptable Träger eine Creme, Lotion, ein Gel oder eine Emulsion ist.

6. Zusammensetzung nach einem der vorstehenden Ansprüche für die Verwendung bei einem Verfahren zum Lichtschutz von Human-Keratinsubstraten,
wobei das Verfahren das Auftragen der Zusammensetzung auf Human-Keratinsubstrate aufweist.

## Revendications

1. Composition d'écran solaire photostable comprenant
(a) de 0,1 % à 10 % en masse de dibenzoylméthane ou de son dérivé ;
(b) de 0,1 % à 10 % en masse d'acide p-méthoxycinnamique ou de son dérivé ;
(c) de 0,5 à 5 % en poids de zingérone ; et
(d) une base cosmétiquement acceptable comprenant un élastomère de silicone ;
dans laquelle le rapport massique de zingérone au dibenzoylméthane est de 0,8 à 2,0.

2. Composition selon la revendication 1, dans laquelle ledit dérivé de dibenzoylméthane est le 4-tert-butyl-4'-méthoxydibenzoyl-méthane.

3. Composition selon la revendication 1, dans laquelle ledit dérivé d'acide p-méthoxycinnamique est le 2-éthyl-hexyl-4-méthoxycinnamate.

4. Composition selon l'une quelconque des revendications précédentes comprenant de 1,0 à 3 % de zingérone en masse de la composition.

5. Composition selon l'une quelconque des revendications précédentes, dans laquelle la base cosmétiquement acceptable est une crème, une lotion, un gel ou une émulsion.

6. Composition selon l'une quelconque des revendications précédentes pour une utilisation dans un procédé de photoprotection de substrats kératineux humains, le procédé comprenant l'application de la composition à des substrats kératineux humains.
